# EUROPÄISCHE PATENTSCHRIFT

(11) **EP 0 433 438 B1**
(45) Veröffentlichungstag und Bekanntmachung des Hinweises auf die Patenterteilung: **16.06.1993**
(21) Anmeldenummer: 90911614.7
(22) Anmeldetag: 09.07.1990
(51) Int. Cl.: A61F 7/10, A61F 7/00

(54) **PACKUNG FÜR DIE THERMOTHERAPIE**
PACKING FOR THERMOTHERAPY
EMBALLAGE POUR THERMOTHERAPIE

(30) Priorität: 10.07.1989 CH 2564/89
(43) Veröffentlichungstag der Anmeldung: 26.06.1991
(73) Patentinhaber: TESCH, Günter, CH-1700 Fribourg (CH)
(72) Erfinder: TESCH, Günter, CH-1700 Fribourg (CH)
(74) Vertreter: Lesser, Karl-Bolko, Dipl.-Ing.
(86) Internationale Anmeldenummer: EP9001109
(87) Internationale Veröffentlichungsnummer: WO9100716

(56) Entgegenhaltungen:
- EP-A- 0 046 894
- EP-A- 0 071 211
- EP-A- 0 276 682
- EP-A-01 625 83

## Beschreibung

Die Erfindung betrifft eine Packung für die Thermotherapie mit einer aus flexibler Folie bestehenden, dicht verschweißten Packungshülle mit einer wärme- bzw. kältespeicherden Flüssigkeit und einem Einlagematerial aus kugelförmigen Teilen.

Eine solche Packung ist z.B. aus der EP-A-0.046.894 bekannt. Das Einlagematerial in dieser Packung besteht aus einer losen Schüttung von geschlossenporigen Schaumstoffkörpern, die sich nicht mit der wärme- bzw. kältespeicherden Flüssigkeit vollsaugen können. Diese Schaumstoffkörper sind Schaumstoffkügelchen aus Polystyrol, die in dieser Form besonders leicht gegeneinander bewegbar sind. Sie weisen einen Durchmesser von 1 bis 5 mm, insbesondere 4 bis 5 mm, auf. Die Schaumstoffkügelchen sind temperaturunempfindlich. Eine mit den Kügelchen gefüllte Packung läßt sich sehr gut an bestimmte Körperstellen modellieren.

In der Praxis zeigt sich jedoch, daß, da die Packung wegen der notwendigen Modellierbarkeit nicht prall mit Schaumstoffkügelchen gefüllt sein darf, diese sich innerhalb der Packung so stark verschieben können, daß sie sich in einem Teil der Packung konzentrieren, während in dem anderen Teil der beutelförmigen Packung keine Schaumstoffkügelchen vorhanden sind. Dies ist erwünscht, so weit die Packung horizontal und eben auf einem Körperteil aufliegt, da dann die Schaumstoffkörper auf der dem Körper abgewandten Seite aufschwimmen und eine Isolierschicht bilden. Dieses Aufschwimmen bewirkt aber dann, wenn die Packung nicht horizontal angeordnet ist, daß sich die Schaumstoffkörper in einem Ende der Packung konzentrieren, wodurch ein über die Fläche der Packung ungleichmäßiges Wärmeverhalten erzielt wird, welches nicht gewünscht ist.

Das Verschieben der Kügelchen in einen Teil der Pakkung tritt schon dann auf, wenn die Packung nicht (flächig) liegend, sondern auf einer Seitenkante stehend gelagert wird. Ein gewolltes Durchmischen des Packungsinhaltes ist sehr schwierig.

Darüberhinaus nehmen die Schaumstoffkügelchen einen sehr großen Teil des Packungsvolumens ein. Die Kügelchen sind jedoch nicht in der Lage, Wärme zu speichern, d.h., die Wärmespeicherung wird allein durch die Flüssigkeit gewährleistet, die jedoch - bei einer nach der Patentanmeldung hergestellten, im Handel erhältlichen Packung - nur etwas mehr als die Hälfte des Packungsvolumens einnimmt. Um nun genauso viel Wärme aufzunehmen bzw. abzugeben, wie andere Packungen, muß eine solche Packung etwa doppelt so dick ausgebildet sein.

In der EP-A-0.046.894 wird darüberhinaus noch eine dort als bekannt vorausgesetzte beutelförmige Packung beschrieben, bei der das Einlagematerial aus einem einteiligen, im wesentlichen flächigen Schaumstoffkörper besteht, der die Flüssigkeit aufsaugt. Dieser Schaumstoffkörper dient im wesentlichen dazu, eine einheitliche Dicke der Packung zu gewährleisten, da die beiden Blätter der beutelförmigen Packung auf diesem Schaumstoffkörper über die gesamte Fläche der Packung aufliegen, wenn die Flüssigkeit im wesentlichen durch den Schaumstoffkörper aufgesaugt wird.

Eine solche Packung läßt sich jedoch nicht sehr gut modellieren, d.h., die Anpassung an die Körperoberfläche ist eingeschränkt. Auch erfolgt die Temperaturabgabe ungehindert, sodaß häufig eine zu starke und rasche Wärmeübertragung auftritt, d.h., der Körperteil wird zu stark gekült oder erwärmt, wobei diese Wirkungen einerseits zu rasch abklingen und andererseits zu unliebsamen Erscheinungen wie Erfrierungen bzw. Verbrennungen führen können.

Der Erfindung liegt die Aufgabe zugrunde, eine gattungsgemäße Packung zu schaffen, die sich nicht nur gut modellieren läßt, sondern bei der auch die Wärmeabgabe bzw. die Wärmeaufnahme langsamer, d.h., über einen längeren Zeitraum, erfolgt.

Diese Aufgabe wird durch eine im Anspruch 1 beschriebene Packung gelöst, bei der die kugelförmigen Teile aus sphärisch verwickelten Fasern gebildete Aggregate sind.

Solche Aggregate werden schon seit Jahren bei der Teppichherstellung (EP-A-0.013.427, EP-A-0.013.427) und neuerdings auch als Daunenersatz in der Bettenindustrie (EP-A-0.257.658, EP-A-0.276.682) benutzt. Während bei den in der Bettenindustrie verwendeten Faseraggregaten eine große Rückfederkraft nach dem Zusammendrücken der Aggregate erwünscht ist, die zwar auch hier vorteilhaft ist, werden hier kompaktere Aggregate verwendet.

Die in der Packung vorhandenen Fasern der Aggregate nehmen trotzdem nur ein geringes Volumen ein, da das Kälte- bzw. Wärmemedium in die Aggregate eindringen und sie durchströmen kann, sodaß fast das gesamte Packungsvolumen für das Kälte- bzw. Wärmemedium ausgenutzt werden kann.

Andererseits hindern die Aggregate das Kälte- bzw. Wärmemedium an einer Konvektionsströmung, wenngleich sie eine solche nicht ausschließen. Die Wärmeleitfähigkeit der Fasern sollte möglichst gering sein.

Durch diese Ausbildung der Packung erfolgt die Wärmeabgabe bzw. -aufnahme relativ langsam, sodaß eine solche Packung über einen längeren Zeitraum auf dem zu behandelnden Körper aufliegen kann.

Vorteilhafterweise weisen die Aggregate synthetische Fasern auf, die keine Reaktion mit dem Medium eingehen können. (Dies können Faser aus Polypropylen sein. ]Werden die Fasern farblich behandelt, so kann der Benutzer auch die Fasern in der Packung erkennen und den Erhaltungszustand der Packung beurteilen.

Gemäß einer bevorzugten Ausführungsform weisen die kugelförmigen Aggregate aus diesen hervorstehende Faserenden auf. Dadurch wird sichergestellt, daß sich die Aggregate nicht gegeneinander verschieben können. Hierzu wird auch auf die EP-A-0.257.658 verwiesen, in der solche speziellen Aggregate beschrieben sind.

Dadurch wird auch verhindert, daß sich das Gemisch aus Feststoff und insbesondere Flüssigkeit in der Packung verschiebt, wenn diese an den Körper anmodelliert wird, da die Aggregate dafür sorgen, daß die beiden die Flüssigkeit einschließenden Folienblätter über die Fläche der Packung gleich weit voneinander entfernt bleiben.

Die Hülle der Packung besteht aus Polyurethan. Dieses ist in dem hier benötigten Temperaturbereich stabil und läßt sich trotzdem gut an einen Körper anmodellieren.

## Patentansprüche

1. Packung für die Thermotherapie, insbesondere Kältetherapie, mit einer aus flexibler Folie bestehenden, dicht verschweißten Packungshülle mit einer wärme- bzw. kältespeicherden Flüssigkeit und einem Einlagematerial aus kugelförmigen Teilen, dadurch gekennzeichnet, daß die kugelförmigen Teile aus sphärisch verwickelten Fasern gebildete Aggregate sind, so daß das Kälte- bzw, Wärmemedium in die Aggregate eindringen und sie durchströmen kann.

2. Packung nach Anspruch 1, dadurch gekennzeichnet, daß die Fasern synthetische, vorzugsweise farblich behandelte Fasern sind.

3. Packung nach einem der vorhergehenden Ansprüche, dadurch gekennzeichnet, daß die kugelförmigen Aggregate aus diesen hervorstehende Faserenden aufweisen.

4. Packung nach einem der vorhergehenden Ansprüche, dadurch gekennzeichnet, daß die Wärmeleitfähigkeit der Fasern der Aggregate wesentlich geringer ist, als die Wärmeleitfähigkeit des Kälte- bzw. Wärmemediums.

5. Packung nach einem der vorhergehenden Ansprüche, dadurch gekennzeichnet, daß die Hülle der Packung aus Polyurethan besteht.

## Revendications

1. Emballage pour la thermothérapie, en particulier frigothérapie, avec une enveloppe d'emballage consistant en un film souple fermé par soudure, avec un liquide conservant la chaleur, respectivement le froid, et un matériau d'insert réalisé à partir d'éléments sphériques, caractérisé en ce que les éléments sphériques se composent d'agrégats formés de fibres entremêlées de forme sphérique, de manière que le fluide frigorifique, respectivement thermique peut pénétrer dans les agrégats et les traverser.

2. Emballage suivant la revendication 1, caractérisé en ce que les fibres sont des fibres synthétiques, de préférence traitées par teinture.

3. Emballage suivant l'une des revendications précédentes, caractérisé en ce que les agrégats sphériques présentent des extrémités de fibre ressortant de ceux-ci.

4. Emballage suivant l'une des revendications précédentes, caractérisé en ce que la conductivité thermique des fibres des agrégats est sensiblement inférieure à la conductivité thermique du fluide frigorifique, respectivement thermique.

5. Emballage suivant l'une des revendications précédentes, caractérisé en ce que l'enveloppe de l'emballage est en polyuréthane.

## Claims

1. Pack for thermo-therapy, in particular cooling therapy, with an enclosing hermetically sealed casing containing a heat-storage or cold-storage liquid and a filling comprising ball-like bodies, characterised in that the ball-like bodies are in the form of aggregates of fibres wound into balls so that the cooling or heating medium can penetrate and flow through the aggregates.

2. Pack according to claim 1 characterised in that the fibres are synthetic fibres, preferably coloured or dyed.

3. Pack according to one of the foregoing claims characterised in that the ball-like aggregates have fibre ends projecting from them.

4. Pack according to one of the foregoing claims characterised in that the thermal conductivity of the fibres of the aggregates is substantially lower than the thermal conductivity of the cooling or heating medium.

5. Pack according to one of the foregoing claims characterised in that the casing of the pack is made of polyurethane.
